# EUROPEAN PATENT APPLICATION

(11) **EP 1 202 072 A2**
(43) Date of publication of application: **02.05.2002**
(21) Application number: 01308916.4
(22) Date of filing: 19.10.2001
(51) Int. Cl.: G01R 33/565

(54) **MRI phase error compensation**

(30) Priority: 30.10.2000 JP 2000329949
(71) Applicant: GE Medical Systems Global Technology Company LLC, Waukesha, Wisconsin 53188 (US)
(72) Inventor: Miyoshi, Mitsuharu, Hino-shi, Tokyo 191-8503 (JP)
(74) Representative: Pedder, James Cuthbert

(57) **Abstract**

For the purpose of restraining residual magnetization, eddy current etc. caused by a phase encoder gradient from affecting an echo that corresponds to that phase encoder gradient, in addition to an echo next to the echo that corresponds to that phase encoder gradient, a corrective component for restraining residual magnetization, eddy current etc. caused by a phase encoder gradient pe from affecting an echo next to an echo that corresponds to that phase encoder gradient pe is divided in two into a pre-corrective component Cpr and a post-corrective component Cpo, and the pre-corrective component Cpr is added to the phase encoder gradient pe and the post-corrective component Cpo is added to a rewinder gradient rw.

## Description

The present invention relates to an MR imaging method, a phase error measuring method and an MRI (magnetic resonance imaging) apparatus, and more particularly to an MR imaging method that can restrain residual magnetization, eddy current etc. caused by a phase encoder gradient from affecting an echo that corresponds to that phase encoder gradient, in addition to an echo next to the echo that corresponds to that phase encoder gradient, a phase error measuring method for measuring a phase error amount caused by a phase encoder gradient, and an MRI apparatus for implementing these methods.

In the publications of Japanese Patent Application Laid Open Nos. H08-322817 and H10-75940, an MR imaging method is disclosed which comprises appending a corrective component for restraining residual magnetization, eddy current etc. caused by a phase encoder gradient from affecting an echo next to an echo that corresponds to that phase encoder gradient, as a corrective pulse to the fore or the rear of either one of the phase encoder gradient and rewinder gradient or dividedly as corrective pulses to the fore and rear of either one of those gradients, or incorporating the corrective component into either one of those gradients.

In the conventional MR imaging method, the residual magnetization, eddy current etc. caused by the phase encoder gradient can be restrained from affecting an echo next to an echo that corresponds to that phase encoder gradient.

However, there is a problem that the method takes no account of the effect the residual magnetization, eddy current etc. caused by the phase encoder gradient has on the echo that corresponds to that phase encoder gradient.

Therefore the present invention seeks to provide an MR imaging method that can restrain at least one of residual magnetization, eddy current etc. caused by a phase encoder gradient from affecting an echo that corresponds to that phase encoder gradient, in addition to an echo next to the echo that corresponds to that phase encoder gradient, a phase error measuring method for measuring a phase error amount caused by a phase encoder gradient, and an MRI apparatus for implementing these methods.

In its first aspect, the present invention provides an MR imaging method using a pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting data of a plurality of echoes, said method characterized in comprising: dividing a corrective component into a pre-corrective component and a post-corrective component, said corrective component being for restraining residual magnetization, eddy current etc. caused by the phase encoder gradient from affecting an echo next to an echo that corresponds to said phase encoder gradient; appending said pre-corrective component as a corrective pulse to the fore or the rear of said phase encoder gradient or dividedly as corrective pulses to the fore and rear of said phase encoder gradient, or incorporating said pre-corrective component into said phase encoder gradient; and appending said post-corrective component as a corrective pulse to the fore or the rear of a corresponding rewinder gradient or dividedly as corrective pulses to the fore and rear of said rewinder gradient, or incorporating said post-corrective component into said rewinder gradient.

According to the MR imaging method in the first aspect, a corrective component for restraining residual magnetization, eddy current etc. caused by a phase encoder gradient from affecting an echo next to an echo that corresponds to that phase encoder gradient is divided in two into a pre-corrective component and a post-corrective component, and the pre-corrective component is added to the phase encoder gradient, and the post-corrective component is added to the rewinder gradient. Therefore, with respect to the echo next to the echo that corresponds to that phase encoder gradient, the correction amount is the same as that before the division, and the effect of the residual magnetization, eddy current etc. can be restrained. Moreover, since the post-corrective component is added to the phase encoder gradient, the effect of the residual magnetization, eddy current etc. at an echo center of the echo that corresponds to that phase encoder gradient is reduced, and the effect on an MR image can be restrained. (Since data at the echo center makes the largest contribution to the quality of an MR image, it is preferable that the effect there be restrained.)

In its second aspect, the present invention provides a phase error measuring method characterized in comprising: transmitting a first echo focusing RF pulse for focusing an echo; applying a phase encoder gradient to a phase axis; applying a rewinder gradient to the phase axis; transmitting a second echo focusing RF pulse; observing a second echo while applying a read gradient to the phase axis; transmitting a third echo focusing RF pulse; observing a third echo while applying a read gradient to the phase axis; and determining a phase error amount caused by said phase encoder gradient from offsets of echo peaks of said second and third echoes.

The effect of the residual magnetization, eddy current etc. caused by the phase encoder gradient appears as an offset of an echo peak from an echo center. The offset of an echo peak from an echo center, however, is difficult to measure because the echo center is difficult to identify when a single echo is employed.

Therefore, according to the phase error measuring method in the second aspect, an echo peak of a second echo and that of a third peak are compared. Since an offset of the echo peak from the echo center of the second echo and an offset of the echo peak from the echo center of the third echo have opposite directions, half of the offsets of the echo peaks of the second and third echoes gives the offset of an echo peak from the echo center. Hence, the phase error amount caused by the phase encoder gradient can be determined from the offset.

In its third aspect, the present invention provides a phase error measuring method characterized in comprising: transmitting a first echo focusing RF pulse for focusing an echo and applying a first crusher gradient to a read axis; applying a phase encoder gradient to a phase axis; applying a rewinder gradient to the phase axis; transmitting a second echo focusing RF pulse and applying a second crusher gradient to the read axis; observing a second echo while applying a read gradient to the phase axis; transmitting a third echo focusing RF pulse and applying a third crusher gradient; observing a third echo while applying a read gradient to the phase axis; and determining a phase error amount caused by said phase encoder gradient from offsets of echo peaks of said second and third echoes.

The effect of the residual magnetization, eddy current etc. caused by the phase encoder gradient appears as an offset of an echo peak from an echo center. The offset of an echo peak from an echo center, however, is difficult to measure because the echo center is difficult to identify when a single echo is employed.

Therefore, according to the phase error measuring method in the third aspect, an echo peak of a second echo and that of a third peak are compared. Since an offset of the echo peak from the echo center of the second echo and an offset of the echo peak from the echo center of the third echo have opposite directions, half of the offsets of the echo peaks of the second and third echoes gives the offset of an echo peak from the echo center. Hence, the phase error amount caused by the phase encoder gradient can be determined from the offset.

Moreover, according to the phase error measuring method in the third aspect, a stimulated echo and an FID (free induction decay) signal, which interfere with the measurement of the phase error amount affecting an echo next to an echo that corresponds to that phase encoder gradient, can be eliminated by crusher gradients.

In its fourth aspect, the present invention provides the MR imaging method of the aforementioned configuration, characterized in: determining said corrective component based on the phase error amount measured by the phase error measuring method of the aforementioned configuration.

While the effect of residual magnetization, eddy current etc. is different among individual MRI apparatuses, since the phase error caused by the residual magnetization, eddy current etc. affecting an echo next to an echo that corresponds to a phase encoder gradient is actually measured to determine the corrective component according to the MR imaging method in the fourth aspect, the best corrective component for each of individual MRI apparatuses can be determined.

In its fifth aspect, the present invention provides the MR imaging method of the aforementioned configuration, characterized in: (1) determining a first corrective component based on the phase error amount measured by the phase error measuring method of the aforementioned configuration; (2) determining a first additional corrective component based on the phase error amount measured by the phase error measuring method of the aforementioned configuration, said method being modified by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient; (3) determining an i-th (i ≥ 1) additional corrective component based on the phase error amount measured by the phase error measuring method of the aforementioned configuration, said method being modified by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient; (4) determining an (i+1)-th corrective component as "said first corrective component + Σ i-th additional corrective component"; and (5) repeating (3) and (4) for i = 1 ― N (≥ 1) to determine an (N+1)-th corrective component as said corrective component.

According to the MR imaging method in the fifth aspect, since actual application of the determined corrective component to further optimize the corrective component is repeated more than one time, the best corrective component for each of individual MRI apparatuses can be determined.

In its sixth aspect, the present invention provides a phase error measuring method characterized in comprising: transmitting a first echo focusing RF pulse for focusing an echo, applying a phase encoder gradient to a phase axis, applying a rewinder gradient to the phase axis, transmitting a second echo focusing RF pulse, observing a second echo while applying a read gradient of positive polarity to the phase axis, transmitting a third echo focusing RF pulse, observing a third echo while applying a read gradient of positive polarity to the phase axis, and determining a first phase error amount Δa caused by said phase encoder gradient from offsets of echo peaks of said second and third echoes; transmitting a first echo focusing RF pulse, applying a phase encoder gradient to the phase axis, applying a rewinder gradient to the phase axis, transmitting a second echo focusing RF pulse, observing a second echo while applying a read gradient of negative polarity to the phase axis, transmitting a third echo focusing RF pulse, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.

As described earlier, the effect of the residual magnetization, eddy current etc. caused by the phase encoder gradient appears as an offset of an echo peak from an echo center. However, the offset is also affected by residual magnetization, eddy current etc. caused by the read gradients applied to the phase axis for observing an echo.

Thus, according to the phase error measuring method in the sixth aspect, observation is performed twice with the polarity of the read gradients inverted. Since the polarity of the read gradients are inverted, the offsets by the effect of the residual magnetization, eddy current etc. caused by these read gradients are in the opposite directions. Hence, by averaging the two observation results, the effect of the residual magnetization, eddy current etc. caused by the read gradients can be canceled out, thereby allowing the phase error amount caused by the phase encoder gradient to be accurately determined.

In its seventh aspect, the present invention provides a phase error measuring method characterized in comprising: transmitting a first echo focusing RF pulse for focusing an echo and applying a first crusher gradient of positive polarity to a read axis, applying a phase encoder gradient to a phase axis, applying a rewinder gradient to the phase axis, transmitting a second echo focusing RF pulse and applying a second crusher gradient of negative polarity to the read axis, observing a second echo while applying a read gradient of positive polarity to the phase axis, transmitting a third echo focusing RF pulse and applying a third crusher gradient of positive polarity, observing a third echo while applying a read gradient of positive polarity to the phase axis, and determining a first phase error amount Δa caused by said phase encoder gradient from offsets of echo peaks of said second and third echoes; transmitting a first echo focusing RF pulse and applying a first crusher gradient of negative polarity to the read axis, applying a phase encoder gradient to the phase axis, applying a rewinder gradient to the phase axis, transmitting a second echo focusing RF pulse and applying a second crusher gradient of positive polarity to the read axis, observing a second echo while applying a read gradient of negative polarity to the phase axis, transmitting a third echo focusing RF pulse and applying a third crusher gradient of negative polarity, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.

As described earlier, the effect of the residual magnetization, eddy current etc. caused by the phase encoder gradient appears as an offset of an echo peak from an echo center. However, the offset is also affected by residual magnetization, eddy current etc. caused by the read gradients applied to the phase axis for observing an echo and the crusher gradients applied to the read axis for eliminating a stimulated echo and an FID signal.

Thus, according to the phase error measuring method in the seventh aspect, observation is performed twice with the polarity of the read gradients and crusher gradients inverted. Since the polarity of the read gradients and crusher gradients are inverted, the offsets by the effect of the residual magnetization, eddy current etc. caused by these gradients are in the opposite directions. Hence, by averaging the two observation results, the effect of the residual magnetization, eddy current etc. caused by the read gradients and crusher gradients can be canceled out, thereby allowing the phase error amount caused by the phase encoder gradient to be accurately determined.

In its eighth aspect, the present invention provides the MR imaging method of the aforementioned configuration, characterized in: determining said corrective component based on the phase error amount measured by the phase error measuring method of the aforementioned configuration.

While the effect of residual magnetization, eddy current etc. is different among individual MRI apparatuses, since the phase error caused by the residual magnetization, eddy current etc. affecting an echo next to an echo that corresponds to a phase encoder gradient is actually measured accurately to determine the corrective component according to the MR imaging method in the eighth aspect, the best corrective component for each of individual MRI apparatuses can be accurately determined.

In its ninth aspect, the present invention provides the MR imaging method of the aforementioned configuration, characterized in: (1) determining a first corrective component based on the phase error amount measured by the phase error measuring method of the aforementioned configuration; (2) determining a first additional corrective component based on the phase error amount measured by the phase error measuring method of the aforementioned configuration, said method being modified by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient; (3) determining an i-th (i ≥ 1) additional corrective component based on the phase error amount measured by the phase error measuring method of the aforementioned configuration, said method being modified by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient; (4) determining an (i+1)-th corrective component as "said first corrective component + Σ i-th additional corrective component"; and (5) repeating (3) and (4) for i = 1 ― N (≥ 1) to determine an (N+1)-th corrective component as said corrective component.

According to the MR imaging method in the ninth aspect, since actual application of the determined corrective component to further optimize the corrective component is repeated more than one times, the best corrective component for each of individual MRI apparatuses can be more accurately determined.

In its tenth aspect, the present invention provides the MR imaging method of the aforementioned configuration, characterized in that said pulse sequence is that of a fast spin echo technique, and said focusing RF pulse is an inversion pulse.

The MR imaging method in the tenth aspect can restrain residual magnetization, eddy current etc. caused by a phase encoder gradient from affecting an echo that corresponds to that phase encoder gradient, in addition to an echo next to the echo that corresponds to that phase encoder gradient, in the fast spin echo technique.

In its eleventh aspect, the present invention provides the phase error measuring method of the aforementioned configuration, characterized in that said focusing RF pulse is an inversion pulse.

According to the phase error measuring method in the eleventh aspect, a phase error caused by a phase encoder gradient in the fast spin echo technique can be suitably measured.

In its twelfth aspect, the present invention provides an MRI apparatus comprising RF pulse transmitting means, gradient pulse applying means and NMR signal receiving means, said apparatus controlling said means to execute a pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting data of a plurality of echoes, said apparatus characterized in further comprising corrective component adding means for: dividing a corrective component into a pre-corrective component and a post-corrective component, said corrective component being for restraining residual magnetization, eddy current etc. caused by the phase encoder gradient from affecting an echo next to an echo that corresponds to said phase encoder gradient; appending said pre-corrective component as a corrective pulse to the fore or the rear of said phase encoder gradient or dividedly as corrective pulses to the fore and rear of said phase encoder gradient, or incorporating said pre-corrective component into said phase encoder gradient; and appending said post-corrective component as a corrective pulse to the fore or the rear of a corresponding rewinder gradient or dividedly as corrective pulses to the fore and rear of said rewinder gradient, or incorporating said post-corrective component into said rewinder gradient.

According to the MRI apparatus in the twelfth aspect, the MR imaging method of the first aspect can be suitably carried out.

In its thirteenth aspect, the present invention provides an MRI apparatus comprising RF pulse transmitting means, gradient pulse applying means and NMR signal receiving means, characterized in further comprising phase error measuring means that controls said means for: transmitting a first echo focusing RF pulse for focusing an echo; applying a phase encoder gradient to a phase axis; applying a rewinder gradient to the phase axis; transmitting a second echo focusing RF pulse; observing a second echo while applying a read gradient to the phase axis; transmitting a third echo focusing RF pulse; observing a third echo while applying a read gradient to the phase axis; and determining a phase error amount caused by said phase encoder gradient from offsets of echo peaks of said second and third echoes.

According to the MRI apparatus in the thirteenth aspect, the phase error measuring method of the second aspect can be suitably carried out.

In its fourteenth aspect, the present invention provides an MRI apparatus comprising RF pulse transmitting means, gradient pulse applying means and NMR signal receiving means, characterized in further comprising phase error measuring means that controls said means for: transmitting a first echo focusing RF pulse for focusing an echo and applying a first crusher gradient to a read axis; applying a phase encoder gradient to a phase axis; applying a rewinder gradient to the phase axis; transmitting a second echo focusing RF pulse and applying a second crusher gradient to the read axis; observing a second echo while applying a read gradient to the phase axis; transmitting a third echo focusing RF pulse and applying a third crusher gradient; observing a third echo while applying a read gradient to the phase axis; and determining a phase error amount caused by said phase encoder gradient from offsets of echo peaks of said second and third echoes.

According to the MRI apparatus in the fourteenth aspect, the phase error measuring method of the third aspect can be suitably carried out.

In its fifteenth aspect, the present invention provides the MRI apparatus of the aforementioned configuration, characterized in comprising the phase error measuring means of the aforementioned configuration, and said phase error measuring means determining the corrective component based on the measured phase error amount.

According to the MRI apparatus in the fifteenth aspect, the MR imaging method of the fourth aspect can be suitably carried out.

In its sixteenth aspect, the present invention provides the MRI apparatus of the aforementioned configuration, characterized in comprising the phase error measuring means of the aforementioned configuration, and said phase error measuring means: (1) determining a first corrective component based on the measured phase error amount; (2) determining a first additional corrective component based on a phase error amount measured by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient; (3) determining an i-th (i ≥ 1) additional corrective component based on a phase error amount measured by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient; (4) determining an (i+1)-th corrective component as "said first corrective component + Σ i-th additional corrective component"; and (5) repeating (3) and (4) for i = 1 ― N (≥ 1) to determine an (N+1)-th corrective component as said corrective component.

According to the MRI apparatus in the sixteenth aspect, the MR imaging method of the fifth aspect can be suitably carried out.

In its seventeenth aspect, the present invention provides an MRI apparatus comprising RF pulse transmitting means, gradient pulse applying means and NMR signal receiving means, characterized in further comprising phase error measuring means that controls said means for: transmitting a first echo focusing RF pulse for focusing an echo, applying a phase encoder gradient to a phase axis, applying a rewinder gradient to the phase axis, transmitting a second echo focusing RF pulse, observing a second echo while applying a read gradient of positive polarity to the phase axis, transmitting a third echo focusing RF pulse, observing a third echo while applying a read gradient of positive polarity to the phase axis, and determining a first phase error amount Δa caused by said phase encoder gradient from offsets of echo peaks of said second and third echoes; transmitting a first echo focusing RF pulse, applying a phase encoder gradient to the phase axis, applying a rewinder gradient to the phase axis, transmitting a second echo focusing RF pulse, observing a second echo while applying a read gradient of negative polarity to the phase axis, transmitting a third echo focusing RF pulse, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.

According to the MRI apparatus in the seventeenth aspect, the MR imaging method of the sixth aspect can be suitably carried out.

In its eighteenth aspect, the present invention provides an MRI apparatus comprising RF pulse transmitting means, gradient pulse applying means and NMR signal receiving means, characterized in comprising phase error measuring means that controls said means for: transmitting a first echo focusing RF pulse for focusing an echo and applying a first crusher gradient of positive polarity to a read axis, applying a phase encoder gradient to a phase axis, applying a rewinder gradient to the phase axis, transmitting a second echo focusing RF pulse and applying a second crusher gradient of negative polarity to the read axis, observing a second echo while applying a read gradient of positive polarity to the phase axis, transmitting a third echo focusing RF pulse and applying a third crusher gradient of positive polarity, observing a third echo while applying a read gradient of positive polarity to the phase axis, and determining a first phase error amount Δa caused by said phase encoder gradient from offsets of echo peaks of said second and third echoes; transmitting a first echo focusing RF pulse and applying a first crusher gradient of negative polarity to the read axis, applying a phase encoder gradient to the phase axis, applying a rewinder gradient to the phase axis, transmitting a second echo focusing RF pulse and applying a second crusher gradient of positive polarity to the read axis, observing a second echo while applying a read gradient of negative polarity to the phase axis, transmitting a third echo focusing RF pulse and applying a third crusher gradient of negative polarity, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.

According to the MRI apparatus in the eighteenth aspect, the MR imaging method of the seventh aspect can be suitably carried out.

In its nineteenth aspect, the present invention provides the MRI apparatus of the aforementioned configuration, characterized in comprising the phase error measuring means of the aforementioned configuration, said phase error measuring means determining the corrective component based on the measured phase error amount.

According to the MRI apparatus in the nineteenth aspect, the MR imaging method of the eighth aspect can be suitably carried out.

In its twentieth aspect, the present invention provides the MRI apparatus of the aforementioned configuration, characterized in comprising the phase error measuring means of the aforementioned configuration, said phase error measuring means: (1) determining a first corrective component based on the measured phase error amount; (2) determining a first additional corrective component based on a phase error amount measured by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient; (3) determining an i-th (i ≥ 1) additional corrective component based on a phase error amount measured by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient; (4) determining an (i+1)-th corrective component as "said first corrective component + Σ i-th additional corrective component"; and (5) repeating (3) and (4) for i = 1 ― N (≥ 1) to determine an (N+1)-th corrective component as said corrective component.

According to the MRI apparatus in the twentieth aspect, the MR imaging method of the ninth aspect can be suitably carried out.

In its twenty-first aspect, the present invention provides the MRI apparatus of the aforementioned configuration, characterized in that said pulse sequence is that of a fast spin echo technique, and said focusing RF pulse is an inversion pulse.

According to the MRI apparatus in the twenty-first aspect, the MR imaging method of the tenth aspect can be suitably carried out.

In its twenty-second aspect, the present invention provides the MRI apparatus of the aforementioned configuration, characterized in that said focusing RF pulse is an inversion pulse.

According to the MRI apparatus in the twenty-second aspect, the MR imaging method of the eleventh aspect can be suitably carried out.

According to the MR imaging method and the MRI apparatus of the present invention, residual magnetization, eddy current etc. caused by a phase encoder gradient can be restrained from affecting an echo that corresponds to that phase encoder gradient, in addition to an echo next to the echo that corresponds to that phase encoder gradient.

Moreover, according the phase error measuring method and the MRI apparatus of the present invention, a phase error amount due to residual magnetization, eddy current etc. affecting an echo next to an echo that corresponds to a phase encoder gradient can be accurately measured.

Further objects and advantages of the present invention will be apparent from the following description of the preferred embodiments of the invention as illustrated in the accompanying drawings.

The invention will now be described in greater detail, by way of example, with reference to the drawings, in which:-
Fig. 1 is a block diagram showing an MRI apparatus in accordance with an embodiment of the present invention.
Fig. 2 is a flow chart showing phase error amount measuring processing in accordance with the present invention.
Fig. 3 is a flow chart continued from Fig. 2.
Fig. 4 is an explanatory diagram showing a phase error measuring pulse sequence in accordance with the present invention.
Fig. 5 is an explanatory diagram showing another phase error measuring pulse sequence in accordance with the present invention.
Fig. 6 is an explanatory diagram showing still another phase error measuring pulse sequence in accordance with the present invention.
Fig. 7 is an explanatory diagram showing still another phase error measuring pulse sequence in accordance with the present invention.
Fig. 8 is a flow chart showing MR imaging scan processing in accordance with one embodiment of the present invention.
Fig. 9 is an explanatory diagram showing one example of an MR imaging pulse sequence in accordance with the present invention.

The present invention will now be described in more detail with reference to embodiments shown in the accompanying drawings. It should be noted that the present invention is not limited to these embodiments.

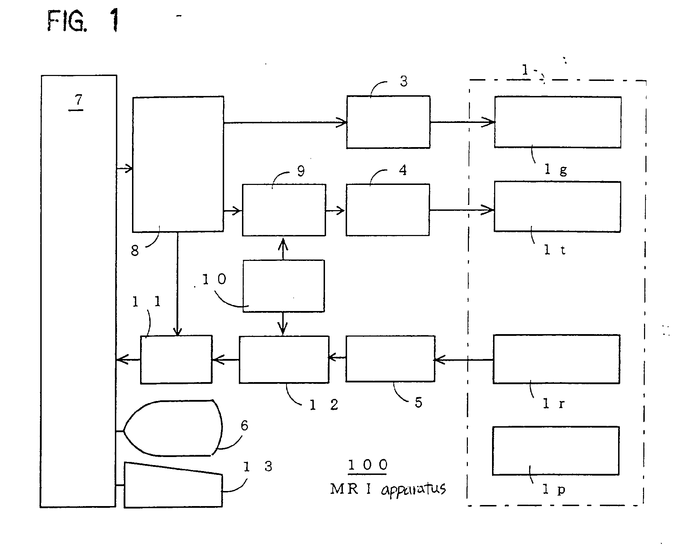

Fig. 1 is a block diagram of an MRI apparatus in accordance with one embodiment of the present invention.

In the MRI apparatus 100, a magnet assembly 1 has an empty portion (bore) therein for inserting a subject, and surrounding the empty portion are disposed a permanent magnet 1p for applying a constant main magnetic field to the subject; a gradient magnetic field coil 1g for generating gradient magnetic fields along X-, Y- and Z-axes; a transmitting coil 1t for supplying an RF pulse to excite spins of atomic nuclei within the subject; and a receiving coil 1r for detecting an NMR signal from the subject. The gradient magnetic field coil 1g, transmitting coil 1t and receiving coil 1r are connected to a gradient magnetic field driving circuit 3, an RF power amplifier 4 and a preamplifier 5, respectively.

It should be noted that a superconductive magnet may be employed instead of the permanent magnet 1p.

A sequence memory circuit 8 operates the gradient magnetic field driving circuit 3 based on a stored pulse sequence in response to instructions from a computer 7, to generate the gradient magnetic fields from the gradient magnetic field coil 1g in the magnet assembly 1. The sequence memory circuit 8 also operates a gate modulation circuit 9 to modulate a carrier output signal from an RF oscillation circuit 10 into a pulsed signal having a predefined timing and envelope shape. The pulsed signal is applied to the RF power amplifier 4 as an RF pulse, power-amplified in the RF power amplifier 4, and applied to the transmitting coil 1t in the magnet assembly 1 to selectively excite a desired imaging plane.

The preamplifier 5 amplifies an NMR signal from the subject detected at the receiving coil 1r in the magnet assembly 1, and inputs the signal to a phase detector 12. The phase detector 12 phase-detects the NMR signal from the preamplifier 5 with reference to the carrier output signal from the RF oscillation circuit 10, and supplies the phase-detected signal to an A/D converter 11. The A/D converter 11 converts the phase-detected analog NMR signal into digital data, and inputs it to the computer 7.

The computer 7 reads the digital data from the A/D converter 11, and performs an image reconstruction operation to produce an MR image of the imaging plane. The computer 7 is also responsible for overall control such as receiving information input from an operating console 13.

A display device 6 displays the MR image.

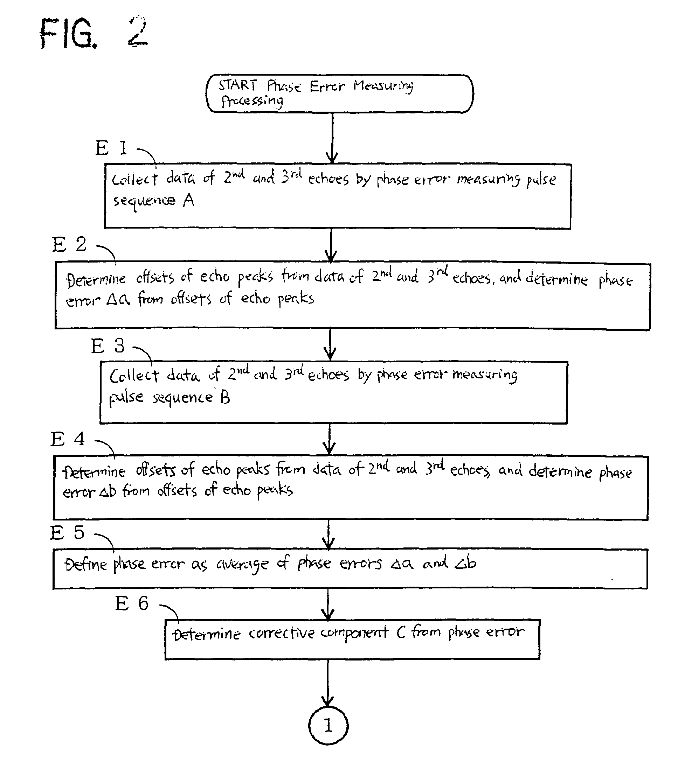

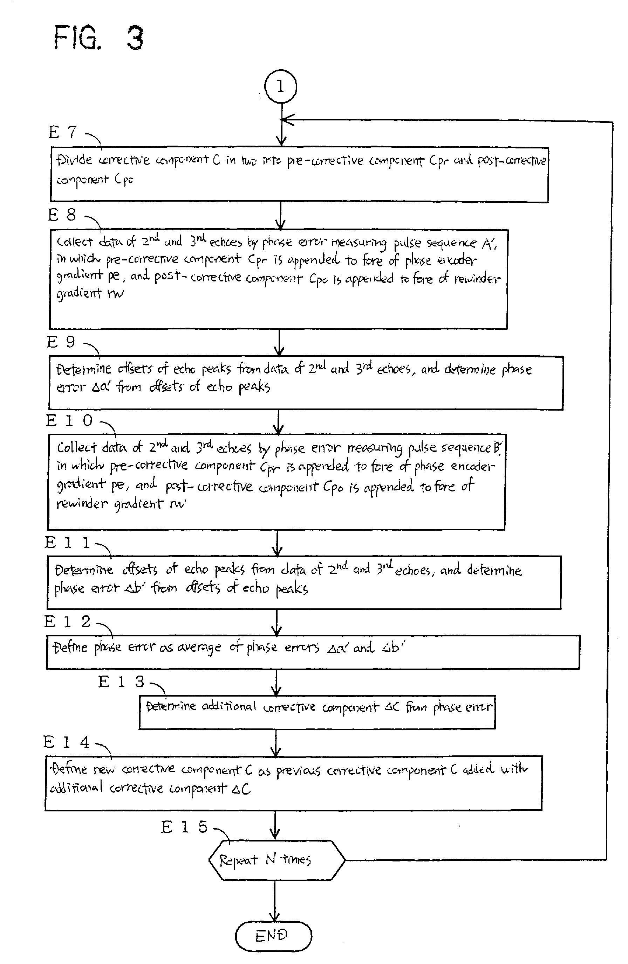

Figs. 2 and 3 are flow charts of phase error amount measuring processing in accordance with the present invention.

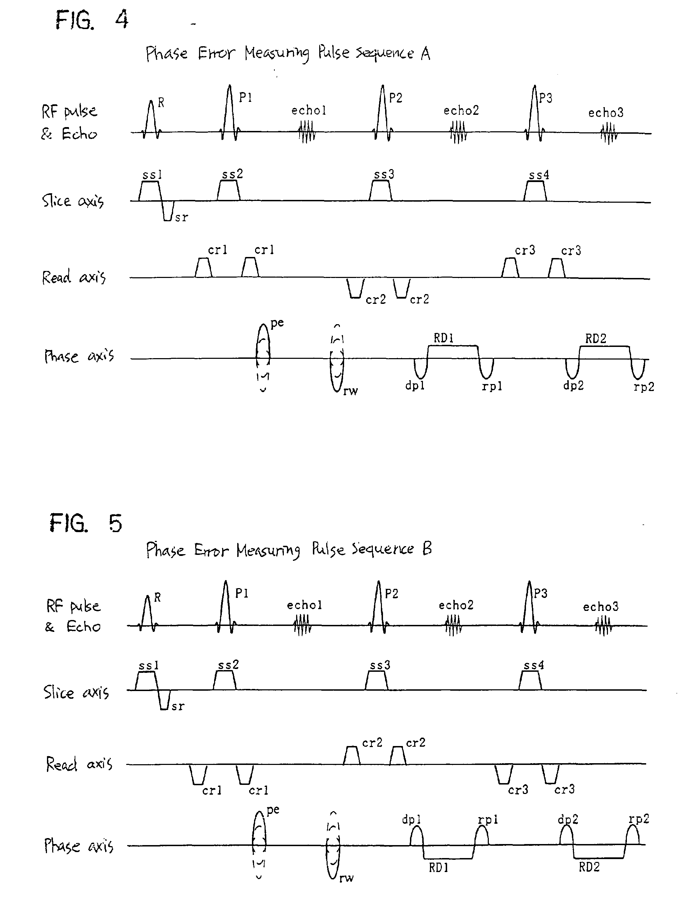

In Step E1 in Fig. 2, data of a second echo echo2 and a third echo echo3 are collected by a phase error measuring pulse sequence A shown in Fig. 4.

In the phase error measuring pulse sequence A of Fig. 4, an excitation pulse R and a slice gradient ss1 are applied. Next, a first inversion pulse P1 and a slice gradient ss2 are applied, and first crusher gradients cr1 of positive polarity are applied to a read axis before and after the first inversion pulse P1. Next, a phase encoder gradient pe is applied to a phase axis, and then, a rewinder gradient is applied to the phase axis.

Next, a second inversion pulse P2 and a slice gradient ss3 are applied, and second crusher gradients cr2 of negative polarity are applied to the read axis before and after the second inversion pulse P2. Next, a dephaser gradient dp1 is applied to the phase axis; next an NMR signal of the second echo echo2 is received while applying a read gradient RD1 to the phase axis; and then, a rephaser gradient rp1 equal to the dephaser gradient dp1 is applied to the phase axis.

Next, a third inversion pulse P3 and a slice gradient ss4 are applied, and third crusher gradients cr3 of positive polarity are applied to the read axis before and after the third inversion pulse P3. Next, a dephaser gradient dp2 is applied to the phase axis; next an NMR signal of the third echo echo3 is received while applying a read gradient RD2 to the phase axis; and then, a rephaser gradient rp2 equal to the dephaser gradient dp2 is applied to the phase axis.

Returning to Fig. 2, in Step E2, offsets of echo peaks are determined from the data of the second echo echo2 and the third echo echo3, and a phase error amount Δa is determined from the offsets of the echo peaks.

In Step E3, data of a second echo echo2 and a third echo echo3 are collected by a phase error measuring pulse sequence B shown in Fig. 5.

The phase error measuring pulse sequence B of Fig. 5 is one in which the polarity of the crusher gradients cr, dephaser gradients dp, read gradients RD and rephaser gradients rp in the phase error measuring pulse sequence A of Fig. 4 are inverted.

In Step E4, offsets of echo peaks are determined from the data of the second echo echo2 and the third echo echo3, and a phase error amount Δb is determined from the offsets of the echo peaks.

In Step E5, a phase error amount is defined by averaging the phase error amount Δa and the phase error amount Δb.

In Step E6, a corrective component C is determined for correcting the phase error amount.

In Step E7 in Fig. 3, the corrective component C is divided in two into a pre-corrective component Cpr and a post-corrective component Cpo. While the ratio of division may basically be 1:1, the ratio may be different from 1:1 depending on the particular MRI apparatus.

In Step E8, data of second and third echoes are collected by a phase error measuring pulse sequence A', in which the pre-corrective component Cpr is appended to the fore of the phase encoder gradient pe, and the post-corrective component Cpo is appended to the fore of the rewinder gradient rw.

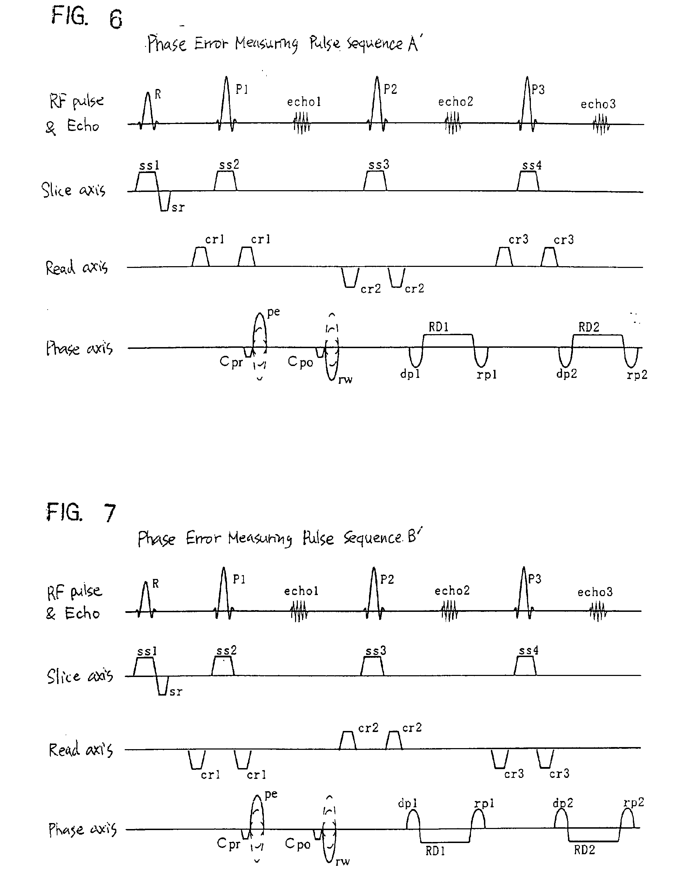

Fig. 6 shows the phase error measuring pulse sequence A'.

In Step E9, offsets of echo peaks are determined from the data of the second echo echo2 and the third echo echo3, and a phase error amount Δa' is determined from the offsets of the echo peaks.

In Step E10, data of second and third echoes are collected by a phase error measuring pulse sequence B', in which the pre-corrective component Cpr is appended to the fore of the phase encoder gradient pe, and the post-corrective component Cpo is appended to the fore of the rewinder gradient rw.

Fig. 7 shows the phase error measuring pulse sequence B'.

In Step E11, offsets of echo peaks are determined from the data of the second echo echo2 and the third echo echo3, and a phase error amount Δb' is determined from the offsets of the echo peaks.

In Step E12, a phase error amount is defined by averaging the phase error amount Δa' and the phase error amount Δb'.

In Step E13, an additional corrective component ΔC is determined for correcting the phase error amount.

In Step E14, a new corrective component C is defined by adding the additional corrective component ΔC to the previous corrective component C.

In Step E15, Steps E7 ― E14 are repeated N (≥ 1) times.

The process is then terminated.

Although the processing of Figs. 2 and 3 may be executed for all of the phase encoder gradients, the number of measurements will be considerably increased. Thus, the processing of Figs. 2 and 3 may be executed for phase encoder gradients after appropriately thinning, and other phase encoder gradients on which the processing of Figs. 2 and 3 is not executed may be made up for by interpolation or extrapolation.

Moreover, although the processing of Figs. 2 and 3 may be executed immediately before performing an MR imaging scan on a patient, the throughput of an MRI apparatus will be reduced. Thus, corrective components determined by executing the processing of Figs. 2 and 3 on patients of different types may be stored into a memory, and a corrective component of a type approximate to a patient to be subjected to an MR imaging scan may be read out of the memory for use.

For simplification, the corrective component C determined by Steps E1 ― E6 in Fig. 2 may be used as determined.

For more simplification, the corrective component C may be determined from the phase error amount Δa determined by Steps E1 and E2 in Fig. 2 for use. Similarly, the corrective component C may be determined from the phase error amount Δb determined by Steps E3 and E4 in Fig. 2 for use.

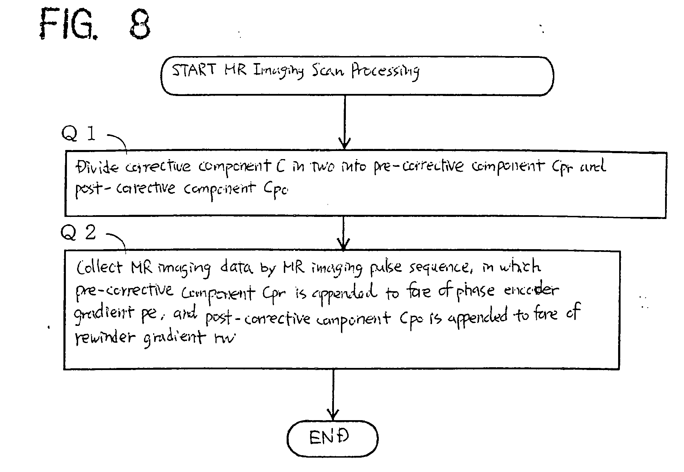

Fig. 8 is a flow chart of MR imaging scan processing.

In Step Q1, a corrective component C determined corresponding to the magnitude of each phase encoder gradient pe is divided in two into a pre-corrective component Cpr and a post-corrective component Cpo. While the ratio of division may basically be 1:1, the ratio may be different from 1:1 depending on an individual MRI apparatus.

In Step Q2, with respect to an MR imaging pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting MR imaging data from a plurality of echoes, the pre-corrective component Cpr is appended to the fore of the phase encoder gradient pe as a corrective pulse Cpr, and the post-corrective component Cpo is appended to the fore of the corresponding rewinder gradient rw as a corrective pulse Cpo to create a new MR imaging pulse sequence, and MR imaging data are collected by the MR imaging pulse sequence.

Then, the process is terminated.

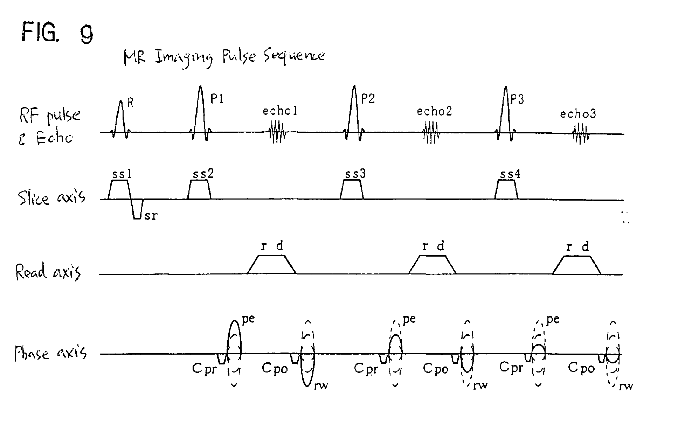

Fig. 9 exemplarily shows an MR imaging pulse sequence in which the present invention is applied to the fast spin echo technique.

It should be noted that the pre-corrective component Cpr may be appended to the rear of the phase encoder gradient pe as a corrective pulse. Similarly, the post-corrective component Cpo may be appended to the rear of the corresponding rewinder gradient rw as a corrective pulse.

Moreover, the pre-corrective component Cpr may be appended dividedly to the fore and rear of the phase encoder gradient pe as two corrective pulses. Similarly, the post-corrective component Cpo may be appended dividedly to the fore and rear of the corresponding rewinder gradient rw as two corrective pulses.

Furthermore, the pre-corrective component Cpr may be incorporated into the area of the phase encoder gradient pe. Similarly, the post-corrective component Cpo may be incorporated into the area of the corresponding rewinder gradient rw.

It should be noted that the present invention can be applied to all MR imaging pulse sequences that generate SSFP (steady-state free precession) signals.

For the sake of good order, various aspects of the invention are set out in the following clauses:-
1. An MR imaging method using a pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting data of a plurality of echoes, said method characterized in comprising:
   dividing a corrective component into a pre-corrective component and a post-corrective component, said corrective component being for restraining residual magnetization, eddy current etc. caused by the phase encoder gradient from affecting an echo next to an echo that corresponds to said phase encoder gradient; appending said pre-corrective component as a corrective pulse to the fore or the rear of said phase encoder gradient or dividedly as corrective pulses to the fore and rear of said phase encoder gradient, or incorporating said pre-corrective component into said phase encoder gradient; and appending said post-corrective component as a corrective pulse to the fore or the rear of a corresponding rewinder gradient or dividedly as corrective pulses to the fore and rear of said rewinder gradient, or incorporating said post-corrective component into said rewinder gradient.
2. A phase error measuring method comprising the steps of: transmitting a first echo focusing RF pulse for focusing an echo; applying a phase encoder gradient to a phase axis; applying a rewinder gradient to the phase axis; transmitting a second echo focusing RF pulse; observing a second echo while applying a read gradient to the phase axis; transmitting a third echo focusing RF pulse; observing a third echo while applying a read gradient to the phase axis; and determining a phase error amount caused by said phase encoder gradient from offsets of echo peaks of said second and third echoes.
3. A phase error measuring method comprising the steps of: transmitting a first echo focusing RF pulse for focusing an echo and applying a first crusher gradient to a read axis; applying a phase encoder gradient to a phase axis; applying a rewinder gradient to the phase axis; transmitting a second echo focusing RF pulse and applying a second crusher gradient to the read axis; observing a second echo while applying a read gradient to the phase axis; transmitting a third echo focusing RF pulse and applying a third crusher gradient; observing a third echo while applying a read gradient to the phase axis; and determining a phase error amount caused by said phase encoder gradient from offsets of echo peaks of said second and third echoes.
4. The MR imaging method of clause 1, wherein: determining said corrective component based on the phase error amount measured by the phase error measuring method of clause 2 or 3.
5. The MR imaging method of clause 1, comprising the steps of:
   (1) determining a first corrective component based on the phase error amount measured by the phase error measuring method of clause 2 or 3;
   (2) determining a first additional corrective component based on the phase error amount measured by the phase error measuring method of clause 2 or 3, said method being modified by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient;
   (3) determining an i-th (i ≥ 1) additional corrective component based on the phase error amount measured by the phase error measuring method of clause 2 or 3, said method being modified by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient;
   (4) determining an (i+1)-th corrective component as "said first corrective component + Σ i-th additional corrective component"; and
   (5) repeating (3) and (4) for i = 1 ― N (≥ 1) to determine an (N+1)-th corrective component as said corrective component.
6. A phase error measuring method comprising the steps of:
   transmitting a first echo focusing RF pulse for focusing an echo, applying a phase encoder gradient to a phase axis, applying a rewinder gradient to the phase axis, transmitting a second echo focusing RF pulse, observing a second echo while applying a read gradient of positive polarity to the phase axis, transmitting a third echo focusing RF pulse, observing a third echo while applying a read gradient of positive polarity to the phase axis, and determining a first phase error amount Δa caused by said phase encoder gradient from offsets of echo peaks of said second and third echoes;
   transmitting a first echo focusing RF pulse, applying a phase encoder gradient to the phase axis, applying a rewinder gradient to the phase axis, transmitting a second echo focusing RF pulse, observing a second echo while applying a read gradient of negative polarity to the phase axis, transmitting a third echo focusing RF pulse, observing a third echo while applying a read gradient of negative polarity to the phase axis, and determining a second phase error amount Δb caused by said phase encoder gradient from offsets of echo peaks of said second and third echoes; and
   averaging said first phase error amount Δa and said second phase error amount Δb to determine a phase error amount.
7. A phase error measuring method characterized in comprising:
   transmitting a first echo focusing RF pulse for focusing an echo and applying a first crusher gradient of positive polarity to a read axis, applying a phase encoder gradient to a phase axis, applying a rewinder gradient to the phase axis, transmitting a second echo focusing RF pulse and applying a second crusher gradient of negative polarity to the read axis, observing a second echo while applying a read gradient of positive polarity to the phase axis, transmitting a third echo focusing RF pulse and applying a third crusher gradient of positive polarity, observing a third echo while applying a read gradient of positive polarity to the phase axis, and determining a first phase error amount Δa caused by said phase encoder gradient from offsets of echo peaks of said second and third echoes;
   transmitting a first echo focusing RF pulse and applying a first crusher gradient of negative polarity to the read axis, applying a phase encoder gradient to the phase axis, applying a rewinder gradient to the phase axis, transmitting a second echo focusing RF pulse and applying a second crusher gradient of positive polarity to the read axis, observing a second echo while applying a read gradient of negative polarity to the phase axis, transmitting a third echo focusing RF pulse and applying a third crusher gradient of negative polarity, observing a third echo while applying a read gradient of negative polarity to the phase axis, and determining a second phase error amount Δb caused by said phase encoder gradient from offsets of echo peaks of said second and third echoes; and
   averaging said first phase error amount Δa and said second phase error amount Δb to determine a phase error amount.
8. The MR imaging method of clause 1, wherein: determining said corrective component based on the phase error amount measured by the phase error measuring method of clause 6 or 7.
9. The MR imaging method of clause 1, comprising the steps of:
   (1) determining a first corrective component based on the phase error amount measured by the phase error measuring method of clause 6 or 7;
   (2) determining a first additional corrective component based on the phase error amount measured by the phase error measuring method of clause 6 or 7, said method being modified by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient;
   (3) determining an i-th (i ≥ 1) additional corrective component based on the phase error amount measured by the phase error measuring method of clause 6 or 7, said method being modified by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient;
   (4) determining an (i+1)-th corrective component as "said first corrective component + Σ i-th additional corrective component"; and
   (5) repeating (3) and (4) for i = 1 ― N (≥ 1) to determine an (N+1)-th corrective component as said corrective component.
10. The MR imaging method of clause 1, 5, or 9, wherein said pulse sequence is that of a fast spin echo technique, and said focusing RF pulse is an inversion pulse.
11. The phase error measuring method of clause 2, 3, 6 or 7, wherein said focusing RF pulse is an inversion pulse.
12. An MRI apparatus comprising an RF pulse transmitting device, a gradient pulse applying device and an NMR signal receiving device, said apparatus controlling said device to execute a pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting data of a plurality of echoes, said apparatus characterized in further comprising:
   a corrective component adding device for dividing a corrective component into a pre-corrective component and a post-corrective component, said corrective component being for restraining residual magnetization, eddy current etc. caused by the phase encoder gradient from affecting an echo next to an echo that corresponds to said phase encoder gradient; appending said pre-corrective component as a corrective pulse to the fore or the rear of said phase encoder gradient or dividedly as corrective pulses to the fore and rear of said phase encoder gradient, or incorporating said pre-corrective component into said phase encoder gradient; and appending said post-corrective component as a corrective pulse to the fore or the rear of a corresponding rewinder gradient or dividedly as corrective pulses to the fore and rear of said rewinder gradient, or incorporating said post-corrective component into said rewinder gradient.
13. An MRI apparatus comprising an RF pulse transmitting device, a gradient pulse applying device and an NMR signal receiving device, characterized in further comprising a phase error measuring device that controls said device for: transmitting a first echo focusing RF pulse for focusing an echo; applying a phase encoder gradient to a phase axis; applying a rewinder gradient to the phase axis; transmitting a second echo focusing RF pulse; observing a second echo while applying a read gradient to the phase axis; transmitting a third echo focusing RF pulse; observing a third echo while applying a read gradient to the phase axis; and determining a phase error amount caused by said phase encoder gradient from offsets of echo peaks of said second and third echoes.
14. An MRI apparatus comprising an RF pulse transmitting device, a gradient pulse applying device and an NMR signal receiving device, characterized in further comprising a phase error measuring device that controls said device for: transmitting a first echo focusing RF pulse for focusing an echo and applying a first crusher gradient to a read axis; applying a phase encoder gradient to a phase axis; applying a rewinder gradient to the phase axis; transmitting a second echo focusing RF pulse and applying a second crusher gradient to the read axis; observing a second echo while applying a read gradient to the phase axis; transmitting a third echo focusing RF pulse and applying a third crusher gradient; observing a third echo while applying a read gradient to the phase axis; and determining a phase error amount caused by said phase encoder gradient from offsets of echo peaks of said second and third echoes.
15. The MRI apparatus of clause 12, characterized in comprising the phase error measuring device of clause 13 or 14, and said phase error measuring device determining the corrective component based on the measured phase error amount.
16. The MRI apparatus of clause 12, characterized in comprising the phase error measuring device of clause 13 or 14, and said phase error measuring device:
   (1) determining a first corrective component based on the measured phase error amount;
   (2) determining a first additional corrective component based on a phase error amount measured by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient;
   (3) determining an i-th (i ≥ 1) additional corrective component based on a phase error amount measured by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient;
   (4) determining an (i+1)-th corrective component as "said first corrective component + Σ i-th additional corrective component"; and
   (5) repeating (3) and (4) for i = 1 - N (≥ 1) to determine an (N+1)-th corrective component as said corrective component.
17. An MRI apparatus comprising an RF pulse transmitting device, a gradient pulse applying device and an NMR signal receiving device, characterized in further comprising a phase error measuring device that controls said device for: transmitting a first echo focusing RF pulse for focusing an echo, applying a phase encoder gradient to a phase axis, applying a rewinder gradient to the phase axis, transmitting a second echo focusing RF pulse, observing a second echo while applying a read gradient of positive polarity to the phase axis, transmitting a third echo focusing RF pulse, observing a third echo while applying a read gradient of positive polarity to the phase axis, and determining a first phase error amount Δa caused by said phase encoder gradient from offsets of echo peaks of said second and third echoes; transmitting a first echo focusing RF pulse, applying a phase encoder gradient to the phase axis, applying a rewinder gradient to the phase axis, transmitting a second echo focusing RF pulse, observing a second echo while applying a read gradient of negative polarity to the phase axis, transmitting a third echo focusing RF pulse, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.
18. An MRI apparatus comprising an RF pulse transmitting device, a gradient pulse applying device and an NMR signal receiving device, characterized in comprising a phase error measuring device that controls said device for: transmitting a first echo focusing RF pulse for focusing an echo and applying a first crusher gradient of positive polarity to a read axis, applying a phase encoder gradient to a phase axis, applying a rewinder gradient to the phase axis, transmitting a second echo focusing RF pulse and applying a second crusher gradient of negative polarity to the read axis, observing a second echo while applying a read gradient of positive polarity to the phase axis, transmitting a third echo focusing RF pulse and applying a third crusher gradient of positive polarity, observing a third echo while applying a read gradient of positive polarity to the phase axis, and determining a first phase error amount Δa caused by said phase encoder gradient from offsets of echo peaks of said second and third echoes; transmitting a first echo focusing RF pulse and applying a first crusher gradient of negative polarity to the read axis, applying a phase encoder gradient to the phase axis, applying a rewinder gradient to the phase axis, transmitting a second echo focusing RF pulse and applying a second crusher gradient of positive polarity to the read axis, observing a second echo while applying a read gradient of negative polarity to the phase axis, transmitting a third echo focusing RF pulse and applying a third crusher gradient of negative polarity, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.
19. The MRI apparatus of clause 12, characterized in comprising the phase error measuring device of clause 17 or 18, said phase error measuring device determining the corrective component based on the measured phase error amount.
20. The MRI apparatus of clause 12, characterized in comprising the phase error measuring device of clause 17 or 18, said phase error measuring device:
   (1) determining a first corrective component based on the measured phase error amount;
   (2) determining a first additional corrective component based on a phase error amount measured by dividing said first corrective component into a first pre-corrective component and a first post-corrective component; appending said first pre-corrective component as a corrective pulse to the fore or the rear of the phase encoder gradient or dividedly as corrective pulses to the fore and rear of said phase encoder gradient, or incorporating said first pre-corrective component into said phase encoder gradient; and appending said first post-corrective component as a corrective pulse to the fore or the rear of the corresponding rewinder gradient or dividedly as corrective pulses to the fore and rear of said rewinder gradient, or incorporating said first post-corrective component into said rewinder gradient;
   (3) determining an i-th (i ≥ 1) additional corrective component based on a phase error amount measured by dividing said i-th corrective component into an i-th pre-corrective component and an i-th post-corrective component; appending said i-th pre-corrective component as a corrective pulse to the fore or the rear of the phase encoder gradient or dividedly as corrective pulses to the fore and rear of said phase encoder gradient, or incorporating said i-th pre-corrective component into said phase encoder gradient; and appending said i-th post-corrective component as a corrective pulse to the fore or the rear of the corresponding rewinder gradient or dividedly as corrective pulses to the fore and rear of said rewinder gradient, or incorporating said i-th post-corrective component into said rewinder gradient;
   (4) determining an (i+1)-th corrective component as "said first corrective component + Σ i-th additional corrective component"; and
   (5) repeating (3) and (4) for i = 1 ― N (≥ 1) to determine an (N+1)-th corrective component as said corrective component.
21. The MRI apparatus of clause 12, 16, or 20, characterized in that said pulse sequence is that of a fast spin echo technique, and said focusing
   RF pulse is an inversion pulse.
22. The MRI apparatus of clause 13, 14, 17 or 18, characterized in that said focusing RF pulse is an inversion pulse.

## Claims

1. An MR imaging method using a pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting data of a plurality of echoes, said method **characterized in** comprising:
dividing a corrective component into a pre-corrective component and a post-corrective component, said corrective component being for restraining residual magnetization, eddy current etc. caused by the phase encoder gradient from affecting an echo next to an echo that corresponds to said phase encoder gradient; appending said pre-corrective component as a corrective pulse to the fore or the rear of said phase encoder gradient or dividedly as corrective pulses to the fore and rear of said phase encoder gradient, or incorporating said pre-corrective component into said phase encoder gradient; and appending said post-corrective component as a corrective pulse to the fore or the rear of a corresponding rewinder gradient or dividedly as corrective pulses to the fore and rear of said rewinder gradient, or incorporating said post-corrective component into said rewinder gradient.

2. A phase error measuring method comprising the steps of: transmitting a first echo focusing RF pulse for focusing an echo; applying a phase encoder gradient to a phase axis; applying a rewinder gradient to the phase axis; transmitting a second echo focusing RF pulse; observing a second echo while applying a read gradient to the phase axis; transmitting a third echo focusing RF pulse; observing a third echo while applying a read gradient to the phase axis; and determining a phase error amount caused by said phase encoder gradient from offsets of echo peaks of said second and third echoes.

3. A phase error measuring method comprising the steps of: transmitting a first echo focusing RF pulse for focusing an echo and applying a first crusher gradient to a read axis; applying a phase encoder gradient to a phase axis; applying a rewinder gradient to the phase axis; transmitting a second echo focusing RF pulse and applying a second crusher gradient to the read axis; observing a second echo while applying a read gradient to the phase axis; transmitting a third echo focusing RF pulse and applying a third crusher gradient; observing a third echo while applying a read gradient to the phase axis; and determining a phase error amount caused by said phase encoder gradient from offsets of echo peaks of said second and third echoes.

4. A phase error measuring method comprising the steps of:
transmitting a first echo focusing RF pulse for focusing an echo, applying a phase encoder gradient to a phase axis, applying a rewinder gradient to the phase axis, transmitting a second echo focusing RF pulse, observing a second echo while applying a read gradient of positive polarity to the phase axis, transmitting a third echo focusing RF pulse, observing a third echo while applying a read gradient of positive polarity to the phase axis, and determining a first phase error amount Δa caused by said phase encoder gradient from offsets of echo peaks of said second and third echoes;
transmitting a first echo focusing RF pulse, applying a phase encoder gradient to the phase axis, applying a rewinder gradient to the phase axis, transmitting a second echo focusing RF pulse, observing a second echo while applying a read gradient of negative polarity to the phase axis, transmitting a third echo focusing RF pulse, observing a third echo while applying a read gradient of negative polarity to the phase axis, and determining a second phase error amount Δb caused by said phase encoder gradient from offsets of echo peaks of said second and third echoes; and
averaging said first phase error amount Δa and said second phase error amount Δb to determine a phase error amount.

5. A phase error measuring method **characterized in** comprising:
transmitting a first echo focusing RF pulse for focusing an echo and applying a first crusher gradient of positive polarity to a read axis, applying a phase encoder gradient to a phase axis, applying a rewinder gradient to the phase axis, transmitting a second echo focusing RF pulse and applying a second crusher gradient of negative polarity to the read axis, observing a second echo while applying a read gradient of positive polarity to the phase axis, transmitting a third echo focusing RF pulse and applying a third crusher gradient of positive polarity, observing a third echo while applying a read gradient of positive polarity to the phase axis, and determining a first phase error amount Δa caused by said phase encoder gradient from offsets of echo peaks of said second and third echoes;
transmitting a first echo focusing RF pulse and applying a first crusher gradient of negative polarity to the read axis, applying a phase encoder gradient to the phase axis, applying a rewinder gradient to the phase axis, transmitting a second echo focusing RF pulse and applying a second crusher gradient of positive polarity to the read axis, observing a second echo while applying a read gradient of negative polarity to the phase axis, transmitting a third echo focusing RF pulse and applying a third crusher gradient of negative polarity, observing a third echo while applying a read gradient of negative polarity to the phase axis, and determining a second phase error amount Δb caused by said phase encoder gradient from offsets of echo peaks of said second and third echoes; and
averaging said first phase error amount Δa and said second phase error amount Δb to determine a phase error amount.

6. An MRI apparatus comprising an RF pulse transmitting device, a gradient pulse applying device and an NMR signal receiving device, said apparatus controlling said device to execute a pulse sequence that involves transmitting an echo focusing RF pulse for focusing an echo, applying a phase encoder gradient to a phase axis, collecting data from an echo while applying a read gradient to a read axis, applying a rewinder gradient to the phase axis, repeating these steps a plurality of times with the phase encoder gradient varied, and successively collecting data of a plurality of echoes, said apparatus **characterized in** further comprising:
a corrective component adding device for dividing a corrective component into a pre-corrective component and a post-corrective component, said corrective component being for restraining residual magnetization, eddy current etc. caused by the phase encoder gradient from affecting an echo next to an echo that corresponds to said phase encoder gradient; appending said pre-corrective component as a corrective pulse to the fore or the rear of said phase encoder gradient or dividedly as corrective pulses to the fore and rear of said phase encoder gradient, or incorporating said pre-corrective component into said phase encoder gradient; and appending said post-corrective component as a corrective pulse to the fore or the rear of a corresponding rewinder gradient or dividedly as corrective pulses to the fore and rear of said rewinder gradient, or incorporating said post-corrective component into said rewinder gradient.

7. An MRI apparatus comprising an RF pulse transmitting device, a gradient pulse applying device and an NMR signal receiving device, **characterized in** further comprising a phase error measuring device that controls said device for: transmitting a first echo focusing RF pulse for focusing an echo; applying a phase encoder gradient to a phase axis; applying a rewinder gradient to the phase axis; transmitting a second echo focusing RF pulse; observing a second echo while applying a read gradient to the phase axis; transmitting a third echo focusing RF pulse; observing a third echo while applying a read gradient to the phase axis; and determining a phase error amount caused by said phase encoder gradient from offsets of echo peaks of said second and third echoes.

8. An MRI apparatus comprising an RF pulse transmitting device, a gradient pulse applying device and an NMR signal receiving device, **characterized in** further comprising a phase error measuring device that controls said device for: transmitting a first echo focusing RF pulse for focusing an echo and applying a first crusher gradient to a read axis; applying a phase encoder gradient to a phase axis; applying a rewinder gradient to the phase axis; transmitting a second echo focusing RF pulse and applying a second crusher gradient to the read axis; observing a second echo while applying a read gradient to the phase axis; transmitting a third echo focusing RF pulse and applying a third crusher gradient; observing a third echo while applying a read gradient to the phase axis; and determining a phase error amount caused by said phase encoder gradient from offsets of echo peaks of said second and third echoes.

9. An MRI apparatus comprising an RF pulse transmitting device, a gradient pulse applying device and an NMR signal receiving device, **characterized in** further comprising a phase error measuring device that controls said device for: transmitting a first echo focusing RF pulse for focusing an echo, applying a phase encoder gradient to a phase axis, applying a rewinder gradient to the phase axis, transmitting a second echo focusing RF pulse, observing a second echo while applying a read gradient of positive polarity to the phase axis, transmitting a third echo focusing RF pulse, observing a third echo while applying a read gradient of positive polarity to the phase axis, and determining a first phase error amount Δa caused by said phase encoder gradient from offsets of echo peaks of said second and third echoes; transmitting a first echo focusing RF pulse, applying a phase encoder gradient to the phase axis, applying a rewinder gradient to the phase axis, transmitting a second echo focusing RF pulse, observing a second echo while applying a read gradient of negative polarity to the phase axis, transmitting a third echo focusing RF pulse, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.

10. An MRI apparatus comprising an RF pulse transmitting device, a gradient pulse applying device and an NMR signal receiving device, **characterized in** comprising a phase error measuring device that controls said device for: transmitting a first echo focusing RF pulse for focusing an echo and applying a first crusher gradient of positive polarity to a read axis, applying a phase encoder gradient to a phase axis, applying a rewinder gradient to the phase axis, transmitting a second echo focusing RF pulse and applying a second crusher gradient of negative polarity to the read axis, observing a second echo while applying a read gradient of positive polarity to the phase axis, transmitting a third echo focusing RF pulse and applying a third crusher gradient of positive polarity, observing a third echo while applying a read gradient of positive polarity to the phase axis, and determining a first phase error amount Δa caused by said phase encoder gradient from offsets of echo peaks of said second and third echoes; transmitting a first echo focusing RF pulse and applying a first crusher gradient of negative polarity to the read axis, applying a phase encoder gradient to the phase axis, applying a rewinder gradient to the phase axis, transmitting a second echo focusing RF pulse and applying a second crusher gradient of positive polarity to the read axis, observing a second echo while applying a read gradient of negative polarity to the phase axis, transmitting a third echo focusing RF pulse and applying a third crusher gradient of negative polarity, observing a third echo while applying a read gradient of negative polarity to the phase axis, and determining a second phase error amount Δb caused by said phase encoder gradient from offsets of echo peaks of said second and third echoes; and averaging said first phase error amount Δa and said second phase error amount Δb to determine a phase error amount.
